(19) Europäisches Patentamt
European Patent Office
Office européen des brevets

(11) Numéro de publication : 0 294 292 B1

(12) # FASCICULE DE BREVET EUROPEEN

(45) Date de publication du fascicule du brevet : **09.10.91 Bulletin 91/41**

(51) Int. Cl.[5] : **C07D 451/12,** A61K 31/46

(21) Numéro de dépôt : **88401341.8**

(22) Date de dépôt : **03.06.88**

(54) **Dérivé de l'acide benzo [B] thiophène - 7 carboxylique, son procédé de préparation et les compositions pharmaceutiques qui le contiennent.**

(30) Priorité : **04.06.87 FR 8707774**

(43) Date de publication de la demande : **07.12.88 Bulletin 88/49**

(45) Mention de la délivrance du brevet : **09.10.91 Bulletin 91/41**

(84) Etats contractants désignés : **AT BE CH DE FR GB IT LI LU NL SE**

(56) Documents cités :
**EP-A- 0 147 044**
**EP-A- 0 189 002**
**WO-A-84/00166**

(73) Titulaire : **ADIR ET COMPAGNIE**
**1 rue Carle Hébert**
**F-92415 Courbevoie Cédex (FR)**

(72) Inventeur : **Malen, Charles**
**3 allée Traversière**
**F-94260 Fresnes (FR)**
Inventeur : **Lacoste, Jean-Michel**
**103 rue Brancas**
**F-92310 Sèvres (FR)**
Inventeur : **Laubie, Michel**
**35 avenue Foch**
**F-92420 Vaucresson (FR)**

**EP 0 294 292 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un nouveau dérivé de l'acide benzo [b] thiophène 7 carboxylique.

La demanderesse a découvert que l'ester de tropanol de cet acide particulier est un antagoniste spécifique particulièrement puissant des récepteurs $5HT_3$, récepteurs que l'on sait impliqués dans de nombreux troubles tant centraux que périphériques.

De façon surprenante, dans la série des esters de tropanyl, parmi les différents isomères de position des acides benzo [b] thiophène — (2, 3, 4, 5, 6, 7) carboxyliques, seul l'isomère en 7 est actif ; de plus, des modifications mineures apportées à cette structure, telle qu'une simple méthylation en 2 ou en 3,annulent pratiquement totalement cette activité. (cf. tableau I)

L'activité antagoniste $5HT_3$ du dérivé de la présente invention est significativement supérieure à celle du composé de référence ICS 205.930, (demande de brevet européen n° 0189002) actuellement en développement clinique. De plus sa durée d'action est significativement plus longue, ce qui le rend plus apte à une application thérapeutique.

L'invention concerne non seulement le composé revendiqué, mais aussi son mode de préparation, ses différents sels d'acide minéraux ou organiques et les compositions pharmaceutiques les contenant.

Plus spécifiquement, la présente invention concerne le dérivé de formule (I) :

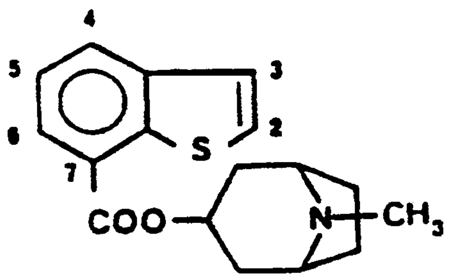

(I)

ou endo [(méthyl-8 aza-8 bicyclo [3.2.1] octyl-3) oxycarbonyl]-7 benzo [b] thiophène, ainsi que les sels d'addition de ce dérivé à un acide pharmaceutiquement acceptable.

Parmi les acides que l'on peut ajouter au composé de formule (I) pour former un sel d'addition, on peut citer, à titre d'exemple, les acides chlorhydrique, sulfurique, phosphorique, tartrique, malique, maléique, fumarique, oxalique, méthanesulfonique, éthanesulfonique, camphorique citrique etc...

La présente invention a également pour objet un procédé de préparation du dérivé de formule (I) caractérisé en ce que l'on condense l' acide benzo [b] thiophène carboxylique-7 de formule (II) :

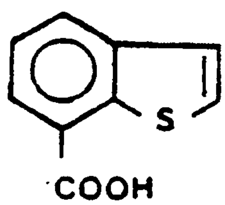

(II)

ou préférentiellement son chlorure, obtenu, par exemple par action sur cet acide du chlorure de thionyle, avec le tropanol-3 ou endo méthyl-8 aza-8 bicyclo [3.2.1] octanol-3 de formule (III) :

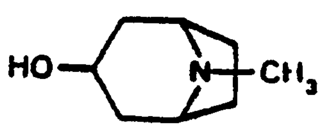

(III)

celui-ci pouvant être préférentiellement utilisé sous forme de sel de métal alcalin, en particulier, sous forme de sel de lithium, un tel sel pouvant être préparé, par exemple, par mélange, sous atmosphère inerte, d'une solution de n butyl lithium dans un solvant inerte tel que l'hexane, à une solution de tropanol-3 dans un solvant inerte tel que le tétrahydrofuranne, pour obtenir le dérivé de formule (I), qui peut être si on le désire, salifié par

un acide pharmaceutiquement acceptable.

Le composé de formule (I) ainsi que ses sels d'addition à un acide pharmaceutiquement acceptable possèdent d'intéressantes propriétés pharmacologiques.

En particulier, le composé de la présente invention montre une activité antagoniste des récepteurs sérotoninergiques 5HT-3 particulièrement intense et dont la durée est étonnamment longue.

Notamment, le composé de la présente invention administré par voie intraveineuse chez le rat, à des doses comprises entre 10 µg/kg et 1 mg/kg antagonise de façon intense et prolongée l'effet Bezold-Jarisch (bradycardie et hypotension après injection de sérotonine). Cette propriété est indicatrice d'un effet antagoniste des récepteurs 5HT-3.

Par conséquent, le composé de la présente invention trouve ses applications thérapeutiques dans le traitement des états découlant d'un dysfonctionnement du système sérotoninergique et dans lesquels le blocage des récepteurs 5HT-3 —, qu'ils soient centraux ou périphériques — est réputé comme pouvant avoir des effets bénéfiques.

Parmi ces états, on peut citer la migraine et plus généralement les épisodes douloureux, l'anxiété, la schizophrénie, les vomissements et notamment ceux consécutifs à l'administration de traitements anticancéreux, les troubles du rythme cardiaque, certains désordres gastro-intestinaux, etc...

L'invention a également pour objet les compositions pharmaceutiques contenant le composé de formule (I) ou un de ses sels d'addition à un acide pharmaceutiquement acceptable, seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration parentérale, per ou transcutanée, nasale, rectale, perlinguale, oculaire ou respiratoire, et notamment les préparations injectables, les aérosols, les gouttes oculaires ou nasales, les capsules, les gélules, les comprimés, les comprimés sublinguaux, les glossettes, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 microgramme et 1 gramme par prise ou par application.

Les exemples suivants illustrent l'invention, et ne la limitent en aucune façon.

Les produits de départ sont connus de la littérature.

Les points de fusion indiqués sont mesurés selon la technique du micro-Köfler. Les spectres de résonance magnétique nucléaire du $^1H$ ont été enregistrés en utilisant le TMS comme référence interne.

**EXEMPLE 1 :** Chlorhydrate d'endo [(méthyl-8 aza-8 bicyclo [3.2.1] octyl-3) oxycarbonyl]-7 benzo [b] thiophène

**STADE A :** Chlorure de l'acide benzo [b] thiophène-7 carboxylique

Une solution de 1,60 g (9 mmol) d'acide benzo [b] thiophène-7 carboxylique et de 13 ml de chlorure de thionyle dans 30 ml de benzène anhydre est chauffée sous reflux pendant 2 heures. Le solvant est ensuite évaporé sous vide puis le résidu est repris dans 30 ml de benzène et la solution à nouveau évaporée (opération répétée 3 fois). La spectroscopie infra-rouge indique que la transformation est totale. Le produit brut de réaction est utilisé tel quel pour la réaction suivante.

**STADE B :** Endo [(méthyl-8 aza-8 bicyclo [3.2.1] octyle-3) oxycarbonyl]-7 benzo [b] thiophène

5,63 ml d'une solution de 9 mmol de n-butyllithium dans l'hexane est additionnée goutte à goutte à une solution refroidie (0°C) de 1,27 g (9 mmol) de tropanol-3 dans 30 ml de tétrahydrofuranne anhydre, sous atmosphère d'azote. Le mélange est agité à cette température pendant 30 minutes ; puis 20 ml d'une solution tétrahydrofurannique du chlorure d'acide obtenu au stade A est additionnée lentement au milieu réactionnel. Après 1 heure d'agitation à 20°C, le milieu réactionnel est évaporé sous vide et le résidu est repris par 50 ml de dichlorométhane. La phase organique est lavée par une solution aqueuse saturée de bicarbonate de potassium puis 3 fois par une solution aqueuse saturée en chlorure de sodium. Après séchage sur sulfate de magnésium et évaporation du solvant, on obtient 2,52 g d'une huile jaune qui, par recristallisation dans un mélange d'éther et d'hexane, permet d'obtenir 1,80 g de prismes incolores du composé désiré.

| | |
|---|---|
| Point de fusion : | 104-105°C |
| Poids moléculaire : | 301, 406 pour $C_{17}H_{19}NO_2S$ |

Composition centésimale :

| | | | | | |
|---|---|---|---|---|---|
| Calculée | : | C : 67,74 | H : 6,35 | N : 4,65 | S : 10,64 |
| Trouvée | : | C : 68,02 | H : 6,40 | N : 4,57 | S : 10,41 |

Caractéristiques spectrales :

Infra rouge (nujol) ν(C = O) : 1695 $cm^{-1}$

RMN $^1H$ ($CDCl_3$)

| | | |
|---|---|---|
| δ (ppm) | 8.20 - 7,80 : | multiplet ; (2H) ; aromatiques $H_4$ - $H_6$ |
| | 7,60 - 7,20 : | multiplet ; (3H) ; aromatiques $H_2$, $H_3$, $H_5$ |
| | 5,50 - 5,20 : | triplet ; (1H) (O - C$\underline{H}$) |
| | 3,30 - 3,00 : | multiplet ; (2H) (C$\underline{H}$ - N - C$\underline{H}$) |
| | 2,30 : | singulet ; (3H) (N - C$\underline{H}_3$) |
| | 2,60 - 1,60 : | multiplet ; (8H) (4 - C$\underline{H}_2$) |

**STADE C :** Endo [(méthyl-8 aza-8 bicyclo [3.2.1] octyle-3) oxy carbonyle]-7 benzyl [b] thiophène chlorhydrate

1,20 g du composé obtenu au stade précédent sont dissous dans 10 ml d'éther anhydre et traités par un équivalent d'une solution d'acide chlorhydrique dans l'éther (3,5 N). Le précipité formé est essoré, lavé par l'éther puis recristallisé dans un mélange d'éthanol et d'éther ; on isole ainsi 1,10 g de prismes incolores du composé désiré, sous forme de chlorhydrate.

Point de fusion : 262-265 °C
Poids moléculaire : 337, 871 pour $C_{17}H_{20}NO_2SCl$

Composition centésimale :

| | | | | | | |
|---|---|---|---|---|---|---|
| Calculée | : | C : 60,43 | H : 5,97 | N : 4,15 | S : 9,49 | Cl : 10,49 |
| Trouvée | : | C : 60,26 | H : 5,89 | N : 4,11 | S : 9,34 | Cl : 10,52 |

Caractéristiques spectrales :

Infra rouge (nujol) ν(C = O) : 1700 $cm^{-1}$
ν($NH^+$) : 2800 - 2300 $cm^{-1}$

RMN $^1H$ ($CDCl_3$)

| | | | |
|---|---|---|---|
| δ (ppm) | 8,30 - 7,90 : | multiplet ; (2H) ; aromatiques $H_4$ - $H_6$ |
| | 7,70 - 7,20 : | multiplet ; 3(H) ; aromatiques $H_2$, $H_3$, $H_5$ |
| | 5,50 : | multiplet ; 1(H) (O - C<u>H</u>) |
| | 4,00- 3,70 : | multiplet ; (2H) (C<u>H</u> - N - C<u>H</u>) |
| | 2,70 : | singulet ; (3H) (N - $C\underline{H}_3$) |
| | 3,60 - 2,00 : | multiplet ; (8H) (4 - $C\underline{H}_2$) |

**EXEMPLE 2 :** Etude pharmacologique du composé de l'invention : inhibition de l'effet Bezold-Jarisch

Cette étude est réalisée sur des rats Charles River d'un poids compris entre 350 et 400 g anesthésiés par une dose de 1,25 g · $kg^{-1}$ d'uréthanne administré intrapéritonéalement. La pression artérielle est enregistrée au niveau d'une artère carotide au moyen d'une cellule de pression Statham P 23 dB. La fréquence cardiaque est enregistrée à partir de la pression artérielle en utilisant un cardiotachymètre. Les enregistrements sont réalisés sur un enregistreur GOULD 2400. Un cathéter est placé dans la veine jugulaire et atteint l'oreillette droite de l'animal. L'effet Bezold-Jarisch est provoqué par une injection rapide de sérotonine à la dose de 40 µg/kg par voie intraveineuse dans l'oreillette droite. Le ralentissement cardiaque est associé à une chute de la pression artérielle qui provient de la stimulation des fibres afférentes vagales principalement localisées dans le ventricule droit.

Les produits à tester sont administrés par voie intraveineuse aux doses de 10 µg/kg, 100 µg/kg et 1 mg/kg. Un effet Bezold-Jarisch est provoqué 5, 15, 30, 45, 60, 90 et 120 minutes après injection des produits testés.

Le pourcentage d'inhibition de la bradycardie réflexe est évalué à partir du tracé de fréquence cardiaque. Les pourcentages d'inhibition provoqués par le produit de l'invention, ses isomères, des produits structuralement voisins, ainsi que par le produit de référence ICS 205930 sont indiqués dans le tableau I qui donne l'inhibition en % du réflexe Bezold chez le rat en fonction du temps ; tous les produits ont été administrés par voie i.v à la même dose de 10 µg/kg. ; les tests ont été réalisés sur 8 animaux.

| Isomères de position A = Tropanyl | Inhibition % après | | | |
|---|---|---|---|---|
| | 15 min. | 30 min. | 60 min. | 120 min. |
| 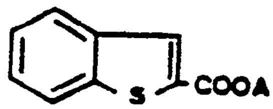 | inactif | inactif | inactif | inactif |
| 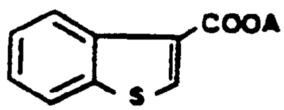 | 38 | 15 | inactif | inactif |
| 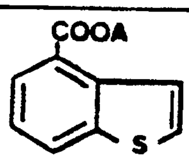 | 20 | inactif | inactif | inactif |
| 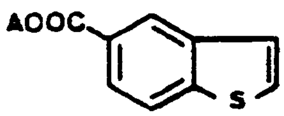 | inactif | inactif | inactif | inactif |
| 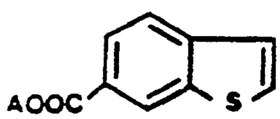 | inactif | inactif | inactif | inactif |
| 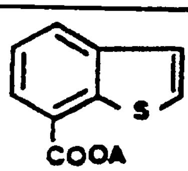 | 86 | 84 | 64 | 47 |
| 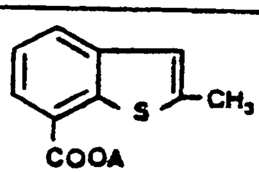 | inactif | inactif | inactif | inactif |
| 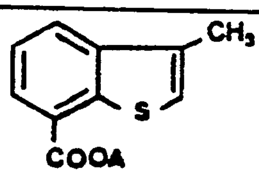 | inactif | inactif | inactif | inactif |
| ICS 205-930 | 79 | 65 | 14 | inactif |

Le produit de la présente invention possède donc un niveau d'activité et une durée d'action particulièrement

surprenants lorsqu'il est comparé :

— à ses isomères de position de la fonction acide carboxylique,

— au composé préféré de l'art antérieur (ICS 205-930).

**EXEMPLE 3 :** Composition pharmaceutique

Formule de préparation pour 1000 comprimés

| | | |
|---|---|---|
| Endo [(Méthyl - 8 aza - 8 bicyclo [3.2.1] octyl - 3) oxycarbonyl] - 7 benzo [b] thiophène, chlorhydrate | 10 | g |
| Hydroxypropylcellulose | 1 | g |
| Amidon de blé | 10 | g |
| Lactose | 100 | g |
| Stéarate de magnésium | 2 | g |
| Talc | 2 | g |

**Revendications**

1. Composé de formule (I) :

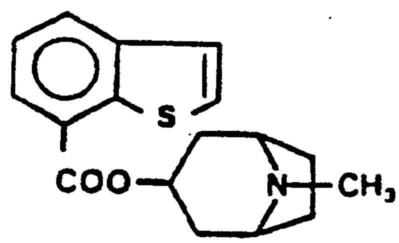

(I)

qui est l'endo [(méthyl-8 aza-8 bicyclo [3.2.1] octyl-3) oxycarbonyl]-7 benzo [b] thiophène ainsi que ses sels d'addition à un acide pharmaceutiquement acceptable.

2. Procédé de préparation du composé de formule (I) caractérisé en ce que l'on condense l'acide benzo [b] thiophène carboxylique-7 de formule (II) :

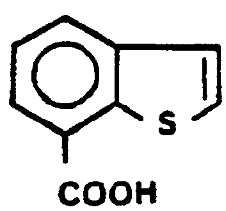

(II)

ou préférentiellement son chlorure obtenu par exemple par action sur cet acide du chlorure de thionyle avec le tropanol-3 de formule (III) :

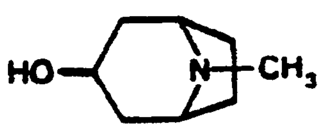

(III)

celui-ci pouvant être préférentiellement utilisé sous forme de sel de métal alcalin en particulier sous forme de

sel de lithium, un tel sel pouvant être préparé par exemple par mélange sous atmosphère inerte d'une solution de n butyllithium dans un solvant inerte tel que l'hexane à une solution de tropanol-3 dans un solvant inerte tel que le tétrahydrofuranne, pour obtenir le dérivé de formule (I) qui peut être si on le désire, salifié par un acide pharmaceutiquement acceptable.

3. Composition pharmaceutique renfermant comme principe actif le composé selon la revendication 1 en combinaison avec un ou plusieurs excipients ou véhicules inertes non toxiques pharmaceutiquement acceptables.

## Patentansprüche

1. Verbindung der Formel (I)

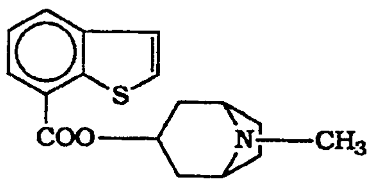

(I)

nämlich endo-7-[(8-Methyl-8-aza-bicyclo[3.2.1]oct-3-yl)-oxycarbonyl]-benzo[b]thiophen sowie dessen Additionssalze mit einer pharmazeutisch annehmbaren Säure.

2. Verfahren zur Herstellung der Verbindung der Formel (I), **dadurch gekennzeichnet**, daß man Benzo[b]thiophen-7-carbonsäure der Formel (II)

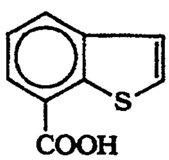

(II)

oder vorzugsweise dessen beispielsweise durch Umsetzen dieser Säure mit Thionylchlorid erhaltenes Chlorid mit Tropanol-3 der Formel (III)

(III)

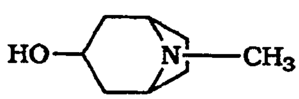

welches man vorzugsweise in Form des Alkalimetallsalzes, insbesondere in Form des Lithiumsalzes, welches man beispielsweise durch Vermischen einer Lösung von Tropanol-3 in einem inerten Lösungsmittel, wie Tetrahydrofuran, mit einer Lösung von n-Butyllithium in einem inerten Lösungsmittel, wie Hexan, unter einer inerten Atmosphäre herstellen kann, verwendet, zur Bildung des Derivats der Formel (I), welches gewünschtenfalls mit einer pharmazeutisch annehmbaren Säure in ein Salz überführt werden kann.

3. Pharmazeutische Zubereitung enthaltend als Wirkstoff die Verbindung nach Anspruch 1 in Kombination mit einem oder mehreren inerten, nichttoxischen, pharmazeutisch annehmbaren Bindemitteln oder Trägermaterialien.

## Claims

1. Compound of the formula (I) :

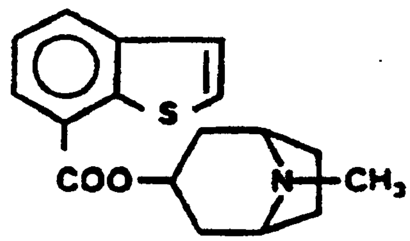

(I)

which is endo-7-(8-methyl-8-azabicyclo[3.2.1]oct-3-yloxycarbonyl)benzo[b]thiophene and also the salts of addition thereof to a pharmaceutically acceptable acid.

2. Method for the preparation of the compound of the formula (I), characterised in that benzo[b]thiophene-7-carboxylic acid of the formula (II) :

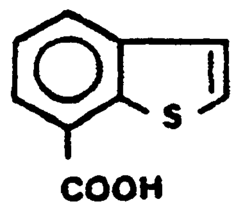

(II)

or preferably its chloride obtained for example by the action on this acid of thionyl chloride, is condensed with tropan-3-ol of the formula (III) :

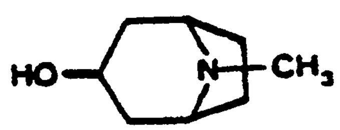

(III)

the latter preferably being used in the form of an alkali metal salt, especially in the form of the lithium salt, it being possible to prepare such a salt for example by mixing under an inert atmosphere a solution of n-butyllithium in an inert solvent such as hexane with a solution of tropan-3-ol in an inert solvent such as tetrahydrofuran, in order to obtain the derivative of the formula (I) which may, if required, be converted into a salt with a pharmaceutically acceptable acid.

3. Pharmaceutical composition containing as active ingredient the compound according to Claim 1 in combination with one or more inert, non-toxic, pharmaceutically acceptable excipients or carriers.